# EUROPEAN PATENT APPLICATION

(11) **EP 4 070 717 A1**
(43) Date of publication of application: **12.10.2022**
(21) Application number: 19955196.1
(22) Date of filing: 06.12.2019
(51) Int. Cl.: A61B 5/00

(54) **METHOD AND DEVICE FOR ESTABLISHING MATHEMATICAL MODEL OF BLOOD VESSEL HAVING STENOTIC LESION**

(30) Priority: 05.12.2019 CN 201911237677
(71) Applicant: Suzhou Rainmed Medical Technology Co., Ltd., Suzhou, Jiangsu 215000 (CN)
(72) Inventor: WANG, Zhiyuan, Suzhou, Jiangsu 215000 (CN); LIU, Guangzhi, Suzhou, Jiangsu 215000 (CN); XU, Lei, Suzhou, Jiangsu 215000 (CN); WANG, Peng, Suzhou, Jiangsu 215000 (CN)
(74) Representative: Boult Wade Tennant LLP
(86) International application number: PCT/CN2019/123659
(87) International publication number: WO 2021/109122

(57) **Abstract**

Disclosed are a method and a device for synthesizing a mathematical model of a blood vessel having a stenotic lesion. The method comprises: performing three-dimensional modeling according to a real-time diameter *Dₜ* of a blood vessel, a length *L* of a blood vessel centerline and a stenotic section to form a three-dimensional model of the blood vessel having a stenotic lesion section (S01); performing N-gon meshing along a circumferential surface of the three-dimensional model of the blood vessel to form a single-layer mesh model (S02); performing surface layering on the single-layer mesh model to form a double-layer mesh model, that is, a mathematical model of the blood vessel (S03). The present disclosure solves the problem of lacking a three-dimensional mesh model of a blood vessel for fluid dynamics analysis in the prior art and fills the gap in the industry. As a blood vessel wall has a certain thickness and a stenosis mainly occurs at an inner wall of the blood vessel, the mathematical model of the blood vessel is established as a double-layer mesh model having a certain thickness, and an inner-layer mesh model of which has elasticity and is able to correct the blood flow velocity, better approximating the actual state of the blood vessel.

## Description

### TECHNICAL FIELD

The present disclosure relates to the field of coronary artery medical technology, and in particular to a method, a device and a system for synthesizing a mathematical model of a blood vessel having a stenotic lesion section for fluid dynamics analysis.

### BACKGROUND

The deposition of lipids and carbohydrates in human blood on a wall of a blood vessel forms plaques on the wall of the blood vessel, then leading to vascular stenosis; especially the stenosis of the blood vessels near the coronary arteries of the heart leads to insufficient blood supply to the heart muscle and induces coronary heart disease, angina pectoris and other diseases, posing a serious threat to human health. According to statistics, there are about 11 million patients suffering from coronary heart diseases in China, and the number of patients treated with cardiovascular interventional surgery is increasing by more than 10% every year.

Although conventional medical detection methods such as coronary angiography (CAG), computed tomography (CT) etc. can display the severity of coronary artery stenosis, they cannot accurately evaluate the situation of coronary ischemia. In order to improve the accuracy of functional evaluation of the coronary artery blood vessel, in 1993, Pijls proposed a new index for calculating the coronary artery blood vessel function through pressure measurement - Fractional Flow Reserve (FFR). After long-term basic and clinical research, FFR has become the gold standard for functional evaluation of coronary stenosis.

FFR is one of the coronary artery blood vessel evaluation parameters, and an index for microcirculatory resistance IMR belongs to the coronary artery blood vessel evaluation parameters.

In coronary artery angiogram images, it is required to combine the fluid dynamics analysis CFD to calculate the coronary artery blood vessel evaluation parameters, but there is no mathematical model of a blood vessel for fluid dynamics analysis in the prior art.

### SUMMARY

The present disclosure provides a method, a device and a system for synthesizing a mathematical model of a blood vessel having a stenotic lesion section for fluid dynamics analysis, so as to solve the problem of lacking a three-dimensional mesh model of a blood vessel for fluid dynamics analysis in the prior art.

In order to achieve the foregoing object, in a first aspect, the present disclosure provides a method for synthesizing a mathematical model of a blood vessel having a stenotic lesion section for fluid dynamics analysis, comprising:
performing three-dimensional modeling according to a real-time diameter *Dₜ* of a blood vessel, a length L of a blood vessel centerline and a stenotic section to form a three-dimensional model of the blood vessel having a stenotic lesion section;
performing N-gon meshing along a circumferential surface of the three-dimensional model of the blood vessel having the stenotic lesion section to form a single-layer mesh model where N≥6;
performing surface layering on the single-layer mesh model to form a double-layer mesh model, that is, a mathematical model of the blood vessel.

Optionally, in the above method for synthesizing a mathematical model of a blood vessel having a stenotic lesion section for fluid dynamics analysis, a manner for performing N-gon meshing along a circumferential surface of the three-dimensional model of the blood vessel having the stenotic lesion section to form a single-layer mesh model where N≥6 comprises:
performing meshing using a triangle as the smallest unit along a circumferential surface of the three-dimensional model of the blood vessel having the stenotic lesion section;
sequentially combining and converting every N triangles into a N-gon to form an initial N-gon mesh;
removing connection lines inside each N-gon in the initial N-gon mesh to form a single-layer N-gon mesh model, where N≥6.

Optionally, in the above method for synthesizing a mathematical model of a blood vessel having a stenotic lesion section for fluid dynamics analysis, a manner for performing meshing using a triangle as the smallest unit along a circumferential surface of the three-dimensional model of the blood vessel having the stenotic lesion section comprises:
segmenting the three-dimensional model of the blood vessel having the stenotic lesion section into K segments,
performing meshing using a triangle as the smallest unit on a circumferential surface of each segment of the three-dimensional model of the blood vessel.

Optionally, in the above method for synthesizing a mathematical model of a blood vessel having a stenotic lesion section for fluid dynamics analysis, the triangle that uses as the smallest unit is an isosceles triangle.

Optionally, in the above method for synthesizing a mathematical model of a blood vessel having a stenotic lesion section for fluid dynamics analysis, a manner for performing surface layering on the single-layer mesh model to form a double-layer mesh model, that is, a mathematical model of the blood vessel, comprises:
acquiring a blood vessel wall thickness h;
performing three-dimensional modeling according to the blood vessel wall thickness h, a starting diameter *D_{starting}* of the blood vessel, an ending diameter *D_{ending}* of the blood vessel and the length L of the blood vessel centerline to form a truncated cone three-dimensional model on an inner surface or an outer surface of the single-layer mesh model;
performing N-gon meshing along a circumferential surface of the truncated cone three-dimensional model according to a manner for acquiring the single-layer mesh model, to form another single-layer mesh model;
forming a double-layer mesh model, that is, a mathematical model of the blood vessel, by busing two layers of the single-layer mesh model and the blood vessel wall thickness h.

Optionally, in the above method for synthesizing a mathematical model of a blood vessel having a stenotic lesion section for fluid dynamics analysis, a manner for performing three-dimensional modeling according to a real-time diameter *Dₜ* of a blood vessel, a length L of a blood vessel centerline and a stenotic section to form a three-dimensional model of the blood vessel having a stenotic lesion section comprises:
acquiring two-dimensional coronary artery angiogram images of at least two body positions;
obtaining a real-time diameter *Dₜ* of the blood vessel and the length L of a straightened blood vessel centerline according to the two-dimensional coronary artery angiogram images;
performing three-dimensional modeling according to the *Dₜ* and L to form a truncated cone three-dimensional model;
acquiring the stenotic section of the coronary artery;
projecting the stenotic section onto the truncated cone three-dimensional model correspondingly to obtain a three-dimensional model of the blood vessel having a stenotic lesion section.

Optionally, in the above method for synthesizing a mathematical model of a blood vessel having a stenotic lesion section for fluid dynamics analysis, a manner for obtaining a real-time diameter *Dₜ* of the blood vessel and the length L of a straightened blood vessel centerline according to the two-dimensional coronary artery angiogram images comprises:
extracting a blood vessel centerline from the two-dimensional coronary artery angiogram image of each body position along a direction from an inlet of the coronary artery to an end of the coronary artery;
obtaining an image of a straightened blood vessel according to the two-dimensional coronary artery angiogram image and the blood vessel centerline;
obtaining a straightened blood vessel contour line according to the straightened blood vessel centerline and the image of the straightened blood vessel;
acquiring geometric information of the straightened blood vessel, comprising: the real-time diameter *Dₜ* of the blood vessel, and the length of the straightened blood vessel centerline, that is, the length L of the centerline of the straightened blood vessel.

Optionally, in the above method for synthesizing a mathematical model of a blood vessel having a stenotic lesion section for fluid dynamics analysis, a manner for performing three-dimensional modeling according to the *Dₜ* and L to form a truncated cone three-dimensional model comprises:
performing three-dimensional modeling according to the geometric information, the centerline and the contour line to obtain a three-dimensional model of the blood vessel having a stenotic lesion section;
acquiring a starting diameter *D_{starting}* of the blood vessel and an ending diameter *D_{ending}* of the blood vessel from the real-time diameter *Dₜ* of the blood vessel;
performing three-dimensional modeling according to the *D_{starting}, D_{ending}* and L to form a truncated cone three-dimensional model.

Optionally, in the above method for synthesizing a mathematical model of a blood vessel having a stenotic lesion section for fluid dynamics analysis, after acquiring two-dimensional coronary artery angiogram images of at least two body positions, and before obtaining a starting diameter *D_{starting}* of the blood vessel and an ending diameter *D_{ending}* of the blood vessel from the real-time diameter *Dₜ* of the blood vessel and the length L of a straightened blood vessel centerline according to the two-dimensional coronary artery angiogram images, further comprising:
acquiring a blood vessel segment of interest from the two-dimensional coronary artery angiogram image;
picking up a starting point and an ending point of the blood vessel segment of interest;
segmenting a blood vessel partial area image corresponding to the starting point and the ending point from the two-dimensional coronary artery angiogram image.

Optionally, in the above method for synthesizing a mathematical model of a blood vessel having a stenotic lesion section for fluid dynamics analysis, a manner for segmenting a blood vessel partial area image corresponding to the starting point and the ending point from the two-dimensional coronary artery angiogram image further comprises:
picking up at least one seed point of the blood vessel segment of interest;
segmenting the two-dimensional angiogram image between two adjacent points of the starting point, the seed point and the ending point, respectively, to obtain at least two blood vessel partial area images.

Optionally, in the above method for synthesizing a mathematical model of a blood vessel having a stenotic lesion section for fluid dynamics analysis, a manner for extracting a blood vessel centerline from the two-dimensional coronary artery angiogram image of each body position along a direction from an inlet of the coronary artery to an end of the coronary artery comprises:
performing image enhancement processing for the blood vessel partial area images to obtain a sharply-contrasting rough image of the blood vessel;
meshing the rough image of the blood vessel, and extracting at least one blood vessel path line along a direction from the starting point to the ending point;
selecting one blood vessel path line as the blood vessel centerline.

Optionally, in the above method for synthesizing a mathematical model of a blood vessel having a stenotic lesion section for fluid dynamics analysis, a manner for meshing the rough image of the blood vessel, and extracting at least one blood vessel path line along a direction from the starting point to the ending point comprises:
meshing the rough image of the blood vessel;
along a blood vessel extension direction from the starting point to the ending point, searching for a point having a shortest path in time with the starting point as a second point from intersecting points of surrounding n meshes, and searching for a point having a shortest path in time with the second point as a third point from intersecting points of surrounding n meshes, and repeating the above step for the third point until the shortest path in time reaches the ending point, where n is a positive integer greater than or equal to 1;
obtaining at least one blood vessel path line by connecting a line extending from the starting point to the ending point according to a searching sequence.

Optionally, in the above method for synthesizing a mathematical model of a blood vessel having a stenotic lesion section for fluid dynamics analysis, a manner for selecting one blood vessel path line as the blood vessel centerline comprises:
summing a time taken for each path line of the blood vessel extending from the starting point to the ending point if there are two or more path lines of the blood vessel;
selecting the path line of the blood vessel with a shortest time as the blood vessel centerline.

Optionally, in the above method for synthesizing a mathematical model of a blood vessel having a stenotic lesion section for fluid dynamics analysis, a manner for extracting a blood vessel centerline from the two-dimensional coronary artery angiogram image of each body position along a direction from an inlet of the coronary artery to an end of the coronary artery comprises:
performing image processing for the blood vessel partial area image to acquire an rough trend line of the blood vessel between the starting point and the ending point;
acquiring a rough edge line of the blood vessel, wherein an image between the rough edge lines of the blood vessel comprising the rough trend line of the blood vessel is a blood vessel skeleton;
extracting the blood vessel centerline from the blood vessel skeleton.

Optionally, in the above method for synthesizing a mathematical model of a blood vessel having a stenotic lesion section for fluid dynamics analysis, a manner for extracting the blood vessel centerline from the blood vessel skeleton comprises:
performing meshing for the blood vessel partial area image after image processing;
along a direction from the starting point to the ending point, according to a RGB value, searching the blood vessel skeleton for a point having a minimum difference in RGB value with the starting point from intersecting points of surrounding m meshes as a second point, searching for a point having a minimum difference in RGB value with the second point from intersecting points of surrounding m meshes as a third point, and repeating the above step for the third point until reaching the ending point, where m is a positive integer greater than or equal to 1;
obtaining at least one connection line from the starting point to the ending point according to a searching sequence;
selecting one connection line as the blood vessel centerline if there are two or more connection lines.

Optionally, in the above method for synthesizing a mathematical model of a blood vessel having a stenotic lesion section for fluid dynamics analysis, a manner for obtaining a straightened blood vessel contour line according to the straightened blood vessel centerline and the image of the straightened blood vessel comprises:
setting a threshold *D_{threshold}* for a diameter of the blood vessel on the image of the straightened blood vessel;
generating a preset contour line of the blood vessel on both sides of a centerline of the straightened blood vessel according to the *D*_{*threshold*;}
making the preset contour line of the blood vessel step-by-step approach the center line of the straightened blood vessel to acquire a straightened blood vessel contour line .

Optionally, in the above method for synthesizing a mathematical model of a blood vessel having a stenotic lesion section for fluid dynamics analysis, a manner for acquiring the stenotic section and the stenotic point comprises:
simulating and generating a smooth curve of a normal blood vessel according to a set extension trend of the normal blood vessel, as well as the real-time diameter *Dₜ* of the blood vessel, the length L of the the centerline of the straightened blood vessel;
comparing the smooth curve of the normal blood vessel generated by simulation with a smooth curve formed by length L-diameter *Dₜ* of a patient's actual centerline of the straightened blood vessel to acquire a stenotic lesion section;
in the stenotic lesion section, picking a point A with the minimum diameter of the smooth curve formed by the length L-diameter *Dₜ* of the patient's actual centerline, and making the point A with the minimum diameter as a stenotic point of the blood vessel segment.

In a second aspect, the present disclosure provides a device for synthesizing a mathematical model of a blood vessel, comprising: a three-dimensional blood vessel model structure, a single-layer mesh model structure and a blood vessel mathematical model structure connected in sequence; the blood vessel mathematical model structure is connected with the three-dimensional model structure;
the three-dimensional blood vessel model structure is configured to perform three-dimensional modeling according to a real-time diameter *Dₜ* of a blood vessel, a length L of a blood vessel centerline and a stenotic section, to form a three-dimensional model of the blood vessel having a stenotic lesion section;
the single-layer mesh model structure is configured to perform N-gon meshing along a circumferential surface of the three-dimensional model of the blood vessel having the stenotic lesion section to form a single-layer mesh model, where N ≥6;
the blood vessel mathematic model structure is configured to perform surface layering for the single-layer mesh model to form a double-layer mesh model, that is, a mathematical model of the blood vessel.

In a third aspect, the present disclosure provides a coronary artery analysis system, comprising: the above device for synthesizing a mathematical model of a blood vessel.

In a fourth aspect, the present disclosure provides a computer storage medium, wherein the above method for synthesizing a mathematical model of a blood vessel having a stenotic lesion section for fluid dynamics analysis is implemented when a computer program is executed by a processor.

The beneficial effects brought about by the solutions provided by the embodiments of the present disclosure comprise at least:
The present disclosure provides a method for synthesizing a mathematical model of a blood vessel having a stenotic lesion section for fluid dynamics analysis, which solves the problem of lacking a three-dimensional mesh model of a blood vessel for fluid dynamics analysis in the prior art and fills the gap in the industry. As a blood vessel wall has a certain thickness and a stenosis mainly occurs at an inner wall of the blood vessel, the mathematical model of the blood vessel is established in the present disclosure as a double-layer mesh model having a certain thickness, an inner-layer mesh model of which has elasticity and is able to correct the blood flow velocity, and an outer-layer mesh model performs the function of shape fixation for the inner-layer mesh model, making the deformation of the inner wall of the blood vessel effectively relieved combined with dynamics analysis, better approximating the actual state of the blood vessel. Further, the minimum unit of the single-layer mesh model is set to a polygon with a number of sides ≥ 6. Due to the poor deformability of a triangle, when one side is impacted by an external force, other sides will also deform, resulting in an relatively large deformation of the triangle, while when a hexagon is impacted by an external force, only two sides deform, and other four sides do not deform, so the deformation of the hexagon is small. Moreover, the double-layer mesh model will form a hexagonal prism, which is more stable than a triangular prism. And the hexagonal has advantages of less sampling points and higher sampling efficiency as compared to the triangle. On the basis of maintaining the original form of the blood vessel, the calculation efficiency for CFD calculation can be effectively improved and the calculation time can be significantly shortened.

### BRIEF DESCRIPTION OF THE DRAWINGS

The drawings illustrated here are used to provide a further understanding of the present disclosure and constitute a part of the present disclosure. The exemplary embodiments and the descriptions thereof are used to explain the present disclosure, and do not constitute an improper limitation on the present disclosure. In the drawings:
FIG. 1 is a structural schematic diagram of a mathematical model of a blood vessel of the present disclosure;
FIG. 2 is a flowchart of a method for synthesizing a mathematical model of a blood vessel having a stenotic lesion section for fluid dynamics analysis of the present disclosure;
FIG. 3 is a structural schematic diagram of a single-layer mesh model of the present disclosure;
FIG. 4 is a flowchart of S02 of the present disclosure;
FIG. 5 is a flowchart of S03 of the present disclosure;
FIG. 6 is a flowchart of S01 of the present disclosure;
FIG. 7 is a flowchart of S400 of the present disclosure;
FIG. 8 is a flowchart of S500 of the present disclosure;
FIG. 9 is a flowchart of the first manner of S510 of the present disclosure;
FIG. 10 is a flowchart of S520 of the present disclosure;
FIG. 11 is a flowchart of a second manner of S510 of the present disclosure;
FIG. 12 is a flowchart of S530' of the present disclosure;
FIG. 13 is a flowchart of S600 of the present disclosure;
FIG. 14 is a flowchart of S700 of the present disclosure;
FIG. 15 is a flowchart of S730 of the present disclosure;
FIG. 16 is a flowchart of S900 of the present disclosure;
FIG. 17 is a three-dimensional model of a blood vessel having a stenotic lesions section;
FIG. 18 is a structural block diagram of a device for synthesizing a mathematical model of a blood vessel;
FIG. 19 is a structural block diagram of a single-layer mesh model structure;
FIG. 20 is a structural block diagram of an embodiment of a three-dimensional blood vessel model structure 1 of the present disclosure;
FIG. 21 is another structural block diagram of an embodiment of a three-dimensional blood vessel model structure 1 of the present disclosure.

### DETAILED DESCRIPTION OF EMBODIMENTS

In order to make objects, technical solutions and advantages of the present disclosure clearer, the technical solutions of the present disclosure will be clearly and completely described below with reference to the specific embodiments and corresponding drawings. It is apparent that the described embodiments are merely part of the embodiments of the present disclosure rather than all of them. Based on the embodiments in the present disclosure, without making creative work, all the other embodiments obtained by a person skilled in the art will fall into the protection scope of the present disclosure.

Hereinafter, a number of embodiments of the present disclosure will be disclosed with drawings. For clear illustration, many practical details will be described in the following description. However, it should be understood that the present disclosure should not be limited by these practical details. In other words, in some embodiments of the present disclosure, these practical details are unnecessary. In addition, in order to simplify the drawings, some conventionally used structures and components will be shown in simple schematic ways in the drawings.

In coronary artery angiogram images, it is required to combine the fluid dynamics analysis CFD to calculate the coronary artery blood vessel evaluation parameters, but there is no mathematical model of a blood vessel for fluid dynamics analysis in the prior art.

### Embodiment 1:

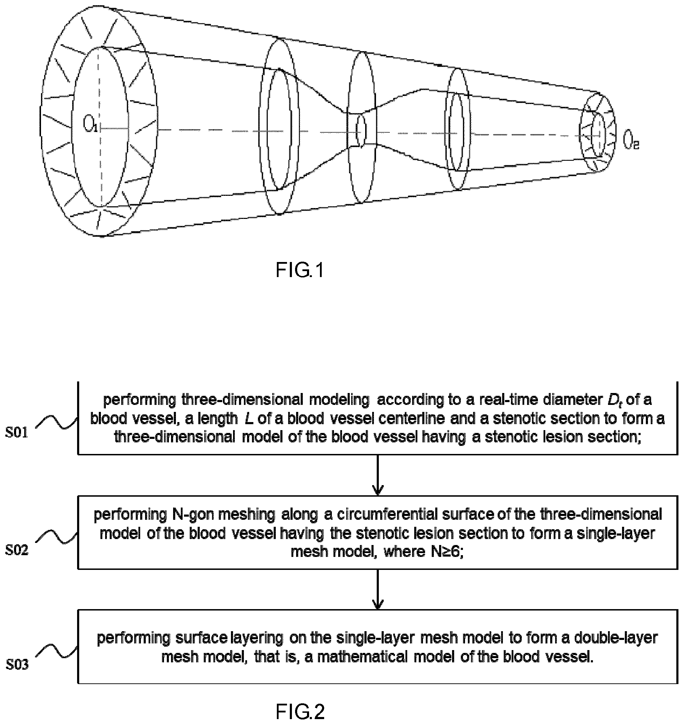

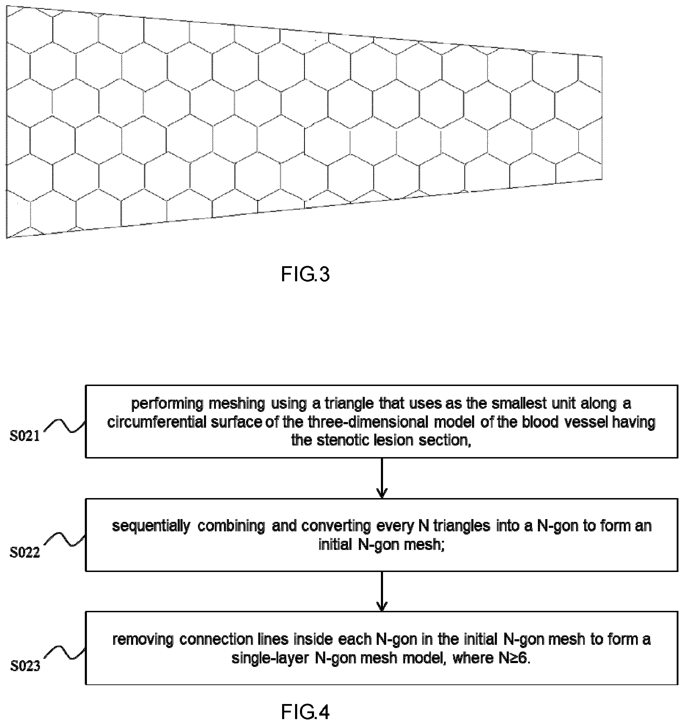

As shown in FIG. 2, the present disclosure provides a method for synthesizing a mathematical model of a blood vessel having a stenotic lesion section for fluid dynamics analysis, comprising:
S01, performing three-dimensional modeling according to a real-time diameter *Dₜ* of a blood vessel, a length L of a blood vessel centerline and a stenotic section to form a three-dimensional model of the blood vessel having a stenotic lesion section;
S02, performing N-gon meshing along a circumferential surface of the three-dimensional model of the blood vessel having the stenotic lesion section to form a single-layer mesh model as shown in FIG. 3, where N≥6;
S03, performing surface layering on the single-layer mesh model to form a double-layer mesh model, that is, a mathematical model of the blood vessel as shown in FIG. 1.

The present disclosure provides a method for synthesizing a mathematical model of a blood vessel having a stenotic lesion section for fluid dynamics analysis, which solves the problem of lacking a three-dimensional mesh model of a blood vessel for fluid dynamics analysis in the prior art and fills the gap in the industry. As a blood vessel wall has a certain thickness and a stenosis mainly occurs at an inner wall of the blood vessel, the mathematical model of the blood vessel is established in the present disclosure as a double-layer mesh model having a certain thickness, better approximating the actual state of the blood vessel, and an outer-layer mesh model performs the function of shape fixation for an inner-layer mesh model, making the deformation of the inner wall of the blood vessel effectively relieved combined with dynamics analysis, better approximating the actual state of the stenosis of the blood vessel. Further, the minimum unit of the single-layer mesh model is set to a polygon with a number of sides ≥ 6. Due to the poor deformability of a triangle, when one side is impacted by an external force, other sides will also deform, resulting in an relatively large deformation of the triangle, while when a hexagon is impacted by an external force, only two sides deform, and other four sides do not deform, so the deformation of the hexagon is small. Moreover, the double-layer mesh model will form a hexagonal prism, which is more stable than a triangular prism. And the hexagonal has advantages of less sampling points and higher sampling efficiency as compared to the triangle. On the basis of maintaining the original form of the blood vessel, the calculation efficiency for CFD calculation can be effectively improved and the calculation time can be significantly shortened.

### Embodiment 2:

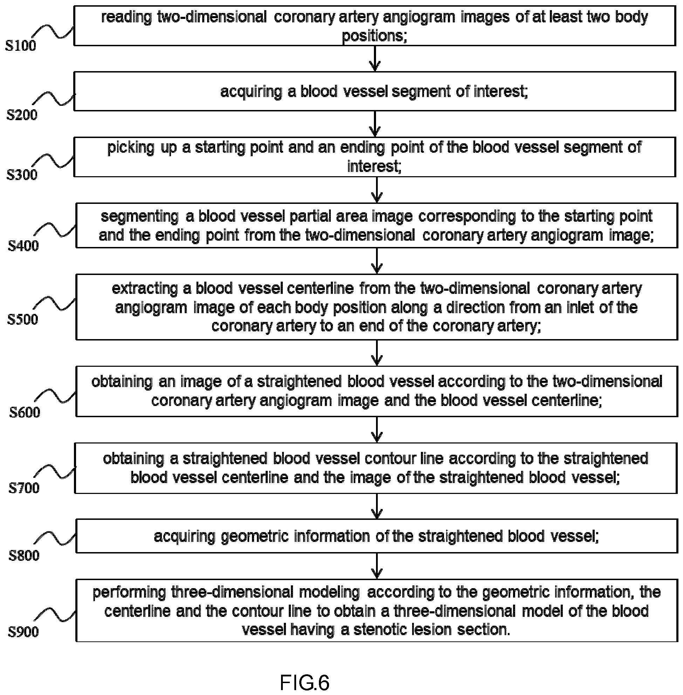

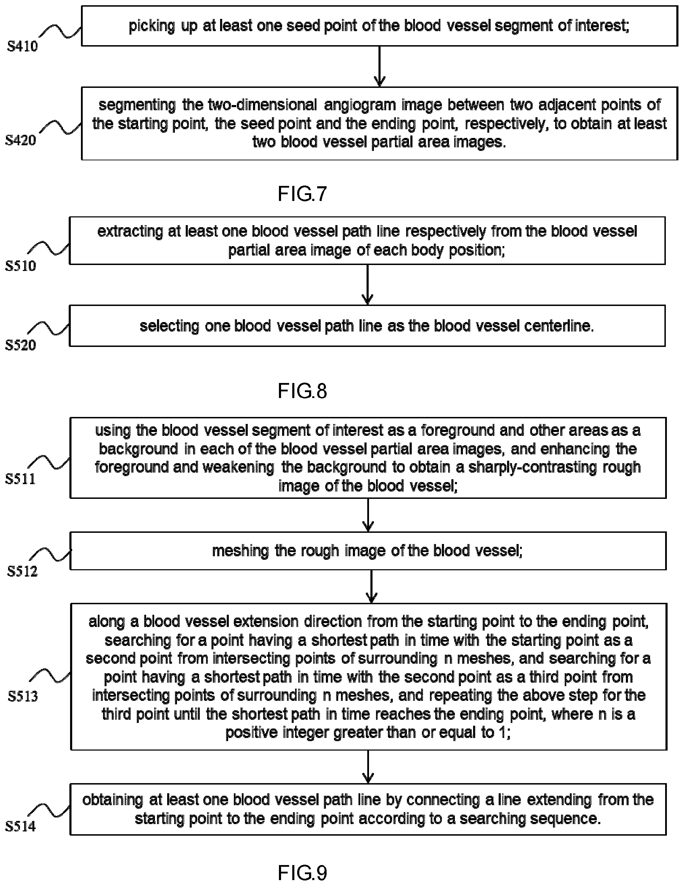

The present disclosure provides a method for synthesizing a mathematical model of a blood vessel having a stenotic lesion section for fluid dynamics analysis, as shown in FIG. 2, comprising:
S01, performing three-dimensional modeling according to a real-time diameter *Dₜ* of a blood vessel, a length L of a blood vessel centerline and a stenotic section to form a three-dimensional model of the blood vessel having a stenotic lesion section, as shown in FIG. 6, comprising:
S100, acquiring two-dimensional coronary artery angiogram images of at least two body positions; preferably, an angle between the two body positions is greater than or equal to 30 degrees;
S200, acquiring a blood vessel segment of interest from the two-dimensional coronary artery angiogram image;
S300, picking up a starting point and an ending point of the blood vessel segment of interest;
S400, segmenting a blood vessel partial area image corresponding to the starting point and the ending point from the two-dimensional coronary artery angiogram image, as shown in FIG. 7, comprising:
   S410, picking up at least one seed point of the blood vessel segment of interest;
   S420, segmenting the two-dimensional angiogram image between two adjacent points of the starting point, the seed point and the ending point, respectively, to obtain at least two blood vessel partial area images;
   S500, obtaining a real-time diameter *Dₜ* of the blood vessel and the length L of a straightened blood vessel centerline according to the two-dimensional coronary artery angiogram images, comprising:
      extracting a blood vessel centerline from the two-dimensional coronary artery angiogram image of each body position along a direction from an inlet of the coronary artery to an end of the coronary artery, comprising two manners.

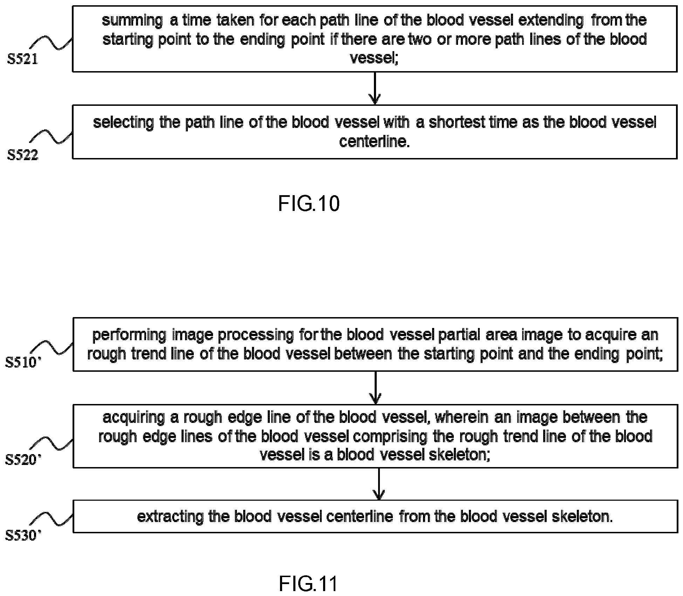

As shown in FIG. 8, a first manner is:
S510, extracting at least one blood vessel path line respectively from the blood vessel partial area image of each body position, as shown in FIG. 9, comprising:
S511, using the blood vessel segment of interest as a foreground and other areas as a background in each of the blood vessel partial area images, and enhancing the foreground and weakening the background to obtain a sharply-contrasting rough image of the blood vessel;
S512, meshing the rough image of the blood vessel;
S513, along a blood vessel extension direction from the starting point to the ending point, searching for a point having a shortest path in time with the starting point as a second point from intersecting points of surrounding n meshes, and searching for a point having a shortest path in time with the second point as a third point from intersecting points of surrounding n meshes, and repeating the above step for the third point until the shortest path in time reaches the ending point, where n is a positive integer greater than or equal to 1;
S514, obtaining at least one blood vessel path line by connecting a line extending from the starting point to the ending point according to a searching sequence;
S520, selecting one blood vessel path line as the blood vessel centerline, as shown in FIG. 10, comprising:
   S521, summing a time taken for each path line of the blood vessel extending from the starting point to the ending point if there are two or more path lines of the blood vessel;
   S522, selecting the path line of the blood vessel with a shortest time as the blood vessel centerline.

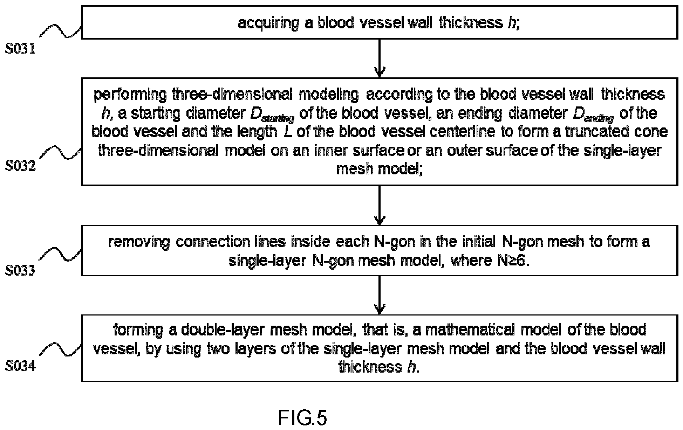

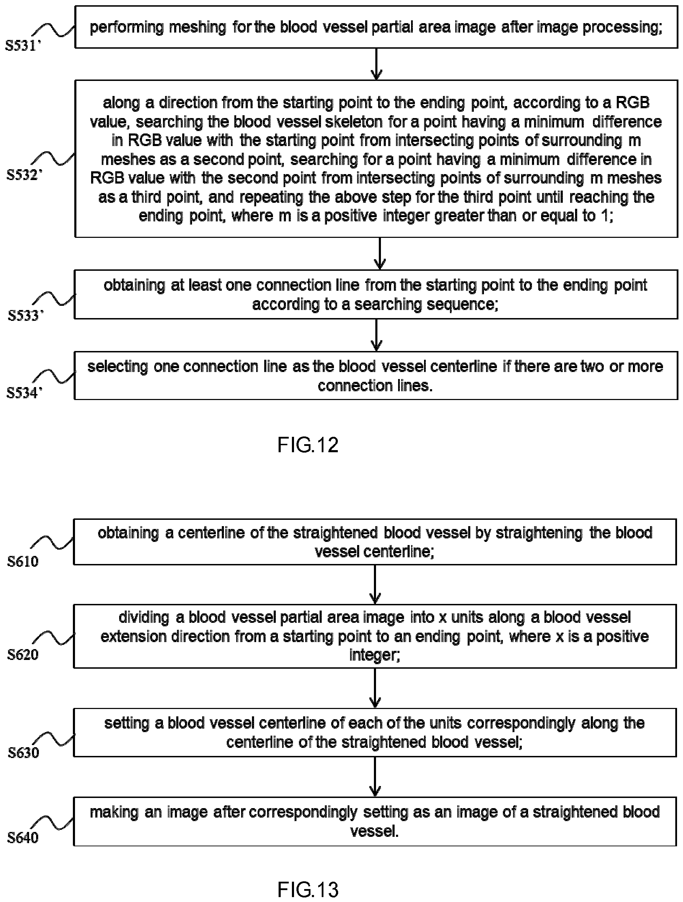

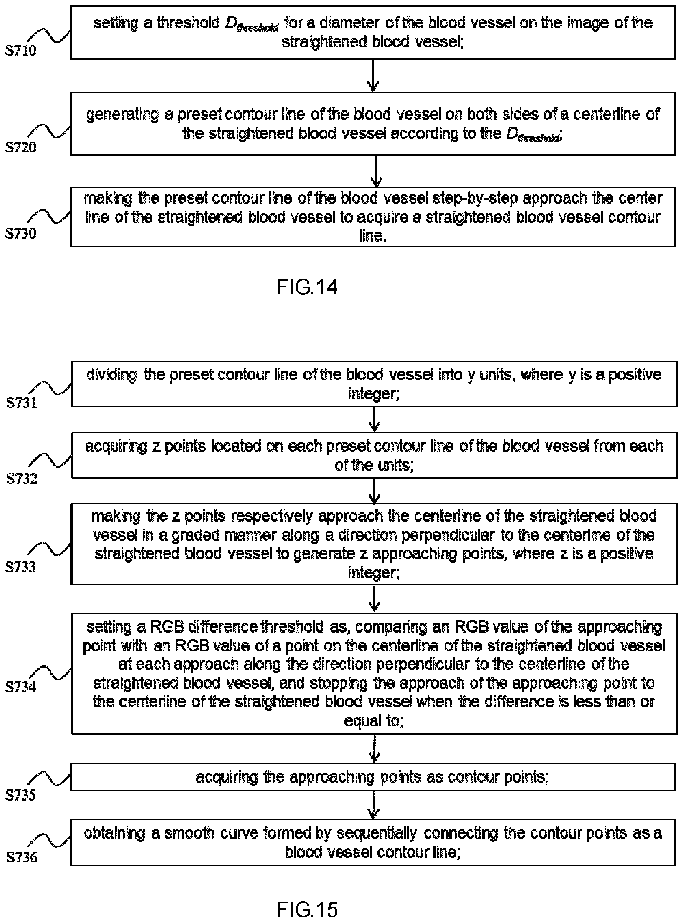

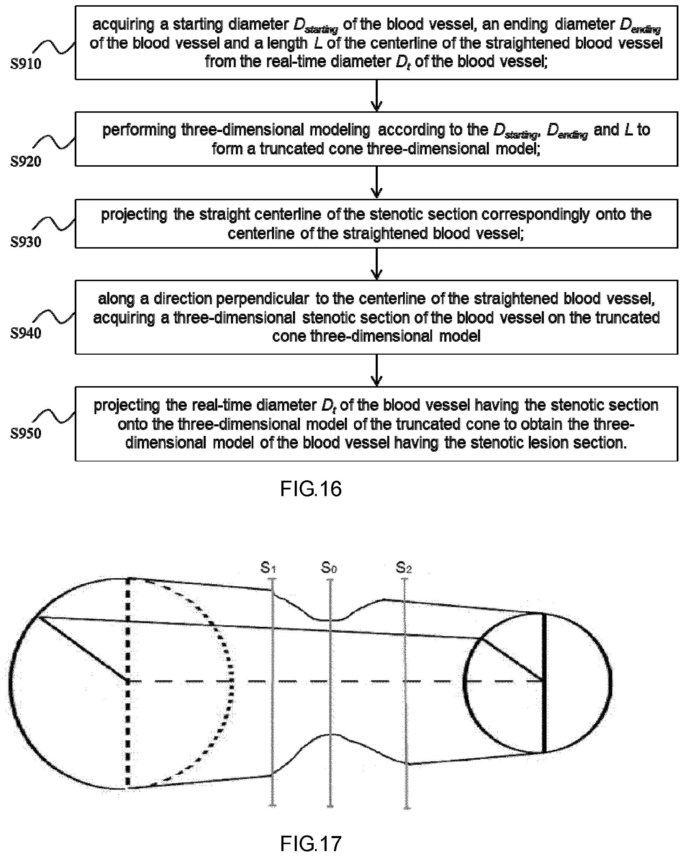

As shown in FIG. 11, a second manner is:
S510', performing image processing for the blood vessel partial area image to acquire an rough trend line of the blood vessel between the starting point and the ending point;
S520', acquiring a rough edge line of the blood vessel, wherein an image between the rough edge lines of the blood vessel comprising the rough trend line of the blood vessel is a blood vessel skeleton;
S530', extracting the blood vessel centerline from the blood vessel skeleton, as shown in FIG. 12, comprising:
   S531', performing meshing for the blood vessel partial area image after image processing;
   S532', along a direction from the starting point to the ending point, according to a RGB value, searching the blood vessel skeleton for a point having a minimum difference in RGB value with the starting point from intersecting points of surrounding m meshes as a second point, searching for a point having a minimum difference in RGB value with the second point from intersecting points of surrounding m meshes as a third point, and repeating the above step for the third point until reaching the ending point, where m is a positive integer greater than or equal to 1;
   S533', obtaining at least one connection line from the starting point to the ending point according to a searching sequence;
   S534', selecting one connection line as the blood vessel centerline if there are two or more connection lines.
   S600, obtaining an image of a straightened blood vessel according to the two-dimensional coronary artery angiogram image and the blood vessel centerline, as shown in FIG. 13, comprising:
      S610, obtaining a centerline of the straightened blood vessel by straightening the blood vessel centerline;
      S620, dividing a blood vessel partial area image into x units along a blood vessel extension direction from a starting point to an ending point, where x is a positive integer;
      S630, setting a blood vessel centerline of each of the units correspondingly along the centerline of the straightened blood vessel;
      S640, making an image after correspondingly setting as an image of a straightened blood vessel;
      S700, obtaining a straightened blood vessel contour line according to the straightened blood vessel centerline and the image of the straightened blood vessel, as shown in FIG. 14, comprising:
         S710, setting a threshold *D_{threshold}* for a diameter of the blood vessel on the image of the straightened blood vessel;
         S720, generating a preset contour line of the blood vessel on both sides of a centerline of the straightened blood vessel according to the *D_{threshold}*;
         S730, making the preset contour line of the blood vessel step-by-step approach the center line of the straightened blood vessel to acquire a straightened blood vessel contour line, as shown in FIG. 15, comprising:
            S731, dividing the preset contour line of the blood vessel into y units, where y is a positive integer;
            S732, acquiring z points located on each preset contour line of the blood vessel from each of the units;
            S733, making the z points respectively approach the centerline of the straightened blood vessel in a graded manner along a direction perpendicular to the centerline of the straightened blood vessel to generate z approaching points, where z is a positive integer;
            S734: setting a RGB difference threshold as *ΔRGB_{threshold},* comparing an RGB value of the approaching point with an RGB value of a point on the centerline of the straightened blood vessel at each approach along the direction perpendicular to the centerline of the straightened blood vessel, and stopping the approach of the approaching point to the centerline of the straightened blood vessel when the difference is less than or equal to *ΔRGB_{threshold}*;
            S735, acquiring the approaching points as contour points;
            S736, obtaining a smooth curve formed by sequentially connecting the contour points as a blood vessel contour line;
            S800, acquiring geometric information of the straightened blood vessel, comprising: the real-time diameter *Dₜ* of the blood vessel, and the length of the straightened blood vessel centerline, that is, the length L of the centerline of the straightened blood vessel, specifically:
               (1) the real-time diameter *Dₜ* of the blood vessel, (2) the stenotic section of the blood vessel, (3) the stenotic point of the blood vessel; (4) length L of the centerline of the straightened blood vessel.
               (1) A manner for acquiring the real-time diameter *Dₜ* of the blood vessel comprises:
                  along a direction perpendicular to the centerline of the straightened blood vessel, acquiring a distance between all oppositely arranged contour points, namely the real-time diameter *Dₜ* of the blood vessel.
               (2) A manner for acquiring the stenotic section of the blood vessel comprises:
                  simulating and generating a smooth curve of a normal blood vessel according to a set extension trend of the normal blood vessel, as well as the real-time diameter *Dₜ* of the blood vessel, the length L of the centerline of the straightened blood vessel;
               comparing the smooth curve of the normal blood vessel generated by simulation with a smooth curve formed by length L-diameter *Dₜ* of a patient's actual centerline of the straightened blood vessel to acquire a stenotic lesion section.
               (3) A manner for acquiring a stenotic point of the blood vessel comprises:
                  in the stenotic lesion section, picking a point A with the minimum diameter of the smooth curve formed by the length L-diameter *Dₜ* of the patient's actual centerline, and making the point A with the minimum diameter as a stenotic point of the blood vessel segment.
            S900, performing three-dimensional modeling according to the *Dₜ* and L to form a truncated cone three-dimensional model, as shown in FIG. 16, comprising:
            S910, acquiring a starting diameter *D_{starting}* of the blood vessel, an ending diameter *D_{ending}* of the blood vessel and a length L of the centerline of the straightened blood vessel from the real-time diameter *Dₜ* of the blood vessel;
            S920, performing three-dimensional modeling according to the *D_{starting}, D_{ending}* and L to form a truncated cone three-dimensional model;
            S014, projecting the stenotic section onto the truncated cone three-dimensional model correspondingly to obtain a three-dimensional model of the blood vessel having a stenotic lesion section, as shown in FIG. 16, comprising:
            S930, projecting the straight centerline of the stenotic section correspondingly onto the centerline of the straightened blood vessel;
            S940, along a direction perpendicular to the centerline of the straightened blood vessel, acquiring a three-dimensional stenotic section S1S2 of the blood vessel on the truncated cone three-dimensional model as shown in FIG. 17;
            S950, projecting the real-time diameter *Dₜ* of the blood vessel having the stenotic section onto the three-dimensional model of the truncated cone to obtain the three-dimensional model of the blood vessel having the stenotic lesion section S1S2 and the stenotic point S0 as shown in FIG. 17;
            S02, performing N-gon meshing along a circumferential surface of the three-dimensional model of the blood vessel having the stenotic lesion section to form a single-layer mesh model where N≥6, as shown in FIG. 4, comprising:
               S021, performing meshing using a triangle as the smallest unit along a circumferential surface of the three-dimensional model of the blood vessel having the stenotic lesion section, comprising:
                  segmenting the three-dimensional model of the blood vessel having the stenotic lesion section into K segments,
                  performing meshing using a triangle as the smallest unit on a circumferential surface of each segment of the three-dimensional model of the blood vessel, and preferably, the triangle that uses as the smallest unit is an isosceles triangle;
               S022, sequentially combining and converting every N triangles into a N-gon to form an initial N-gon mesh;
               S023, removing connection lines inside each N-gon in the initial N-gon mesh to form a single-layer N-gon mesh model, where N≥6;
               S03, performing surface layering on the single-layer mesh model to form a double-layer mesh model, that is, a mathematical model of the blood vessel, as shown in FIG. 5, comprising:
               S031, acquiring a blood vessel wall thickness h; preferably, *h*=0.2mm~ 2mm;
               S032, performing three-dimensional modeling according to the blood vessel wall thickness *h*, a starting diameter *D_{starting}* of the blood vessel, an ending diameter *D_{ending}* of the blood vessel and the length *L* of the blood vessel centerline to form a truncated cone three-dimensional model on an inner surface or an outer surface of the single-layer mesh model;
               S033, performing N-gon meshing along a circumferential surface of the truncated cone three-dimensional model according to a manner for acquiring the single-layer mesh model, to form another single-layer mesh model;
               S034, forming a double-layer mesh model, that is, a mathematical model of the blood vessel, by using two layers of the single-layer mesh model and the blood vessel wall thickness *h*.

### Embodiment 3:

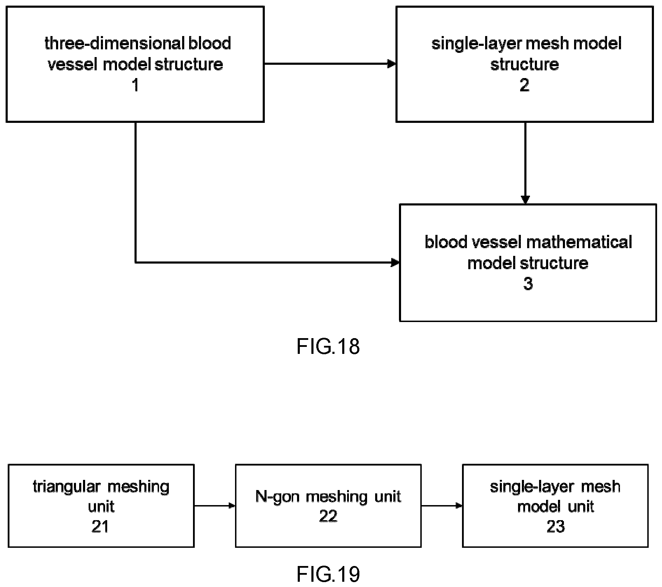

As shown in FIG. 18, the present disclosure provides a device for synthesizing a mathematical model of a blood vessel, comprising: a three-dimensional blood vessel model structure 1, a single-layer mesh model structure 2 and a blood vessel mathematical model structure 3 connected in sequence; the blood vessel mathematical model structure 3 is connected with the three-dimensional model structure 1. The three-dimensional blood vessel model structure 1 is configured to perform three-dimensional modeling according to a real-time diameter *Dₜ* of a blood vessel, a length L of a blood vessel centerline and a stenotic section, to form a three-dimensional model of the blood vessel having a stenotic lesion section. The single-layer mesh model structure 2 is configured to perform N-gon meshing along a circumferential surface of the three-dimensional model of the blood vessel having the stenotic lesion section to form a single-layer mesh model, where N≥6. The blood vessel mathematic model structure 3 is configured to perform surface layering for the single-layer mesh model to form a double-layer mesh model, that is, a mathematical model of the blood vessel.

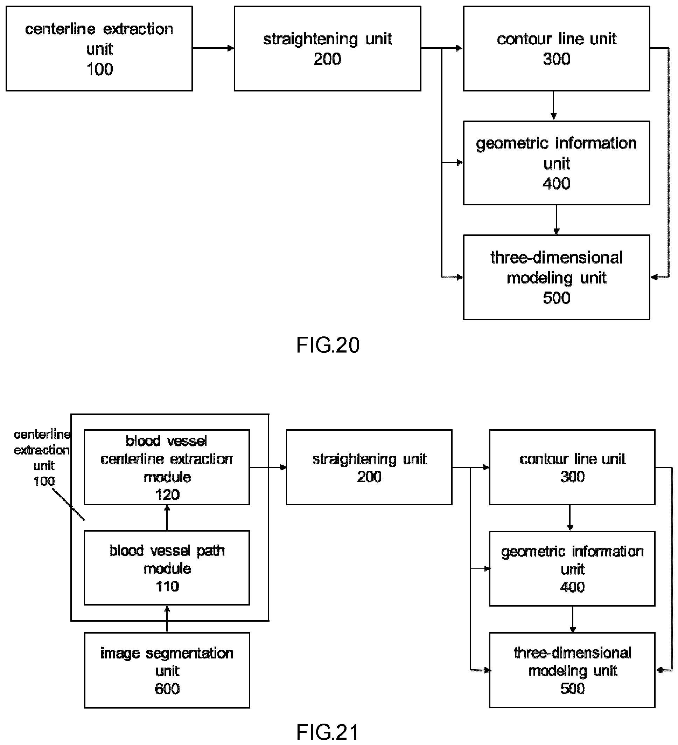

As shown in FIG. 20, the three-dimensional blood vessel model structure 1 further comprises: a centerline extraction unit 100, a straightening unit 200, a contour line unit 300, a geometric information unit 400 and a three-dimensional modeling unit 500 connected in sequence. The straightening unit 200 is connected with the geometric information unit 400. The three-dimensional modeling unit 500 is connected with the straightening unit 200 and the contour line unit 300. The centerline extraction unit 100 is configured to extract a blood vessel centerline respectively from coronary artery angiogram images of at least two body positions along a direction from an inlet of the coronary artery to an end of the coronary artery. The straightening unit 200 is configured to receive the blood vessel centerline sent by the centerline extraction unit 100, and to acquire an image of a straightened blood vessel according to the two-dimensional coronary artery angiogram images and the blood vessel centerline. The contour line unit 300 is configured to receive the image of the straightened blood vessel sent by the straightening unit 200, and to acquire a straightened blood vessel contour line according to a straightened blood vessel centerline and the image of the straightened blood vessel. The geometric information unit 400 is configured to receive the image of the straightened blood vessel sent by the straightening unit 200 and the blood vessel contour line sent by the contour line unit 300, to acquire geometric information of the straightened blood vessel. The three-dimensional modeling unit 500 is configured to receive the image of the straightened blood vessel sent by the straightening unit 200 and the blood vessel contour line sent by the contour line unit 300, the geometric information of the blood vessel sent by the geometric information unit 400, and to perform three-dimensional modeling according to the geometric information, the centerline and the contour line to obtain a three-dimensional model of the blood vessel having a stenotic lesion section.

As shown in FIG. 21, in an embodiment of the present disclosure, the above device further comprises: an image segmentation unit 600 connected to the centerline extraction unit 100. The image segmentation unit 600 is configured to segment a blood vessel partial area image corresponding to the starting point and the ending point from the two-dimensional coronary artery angiogram image, or segmente the two-dimensional angiogram image between two adjacent points of the starting point, the seed point and the ending point to obtain at least two blood vessel partial area images.

As shown in FIG. 21, in an embodiment of the present disclosure, the centerline extraction unit 100 further comprises: a blood vessel path module 110 and a blood vessel centerline extraction module 120 connected in sequence. The blood vessel path module 110 is connected with the image segmentation unit 600. The blood vessel path module 110 is configured to extract at least one blood vessel path line respectively from the blood vessel partial area image of each body position. The blood vessel centerline extraction module 120 is configured to select one of the blood vessel path lines sent by the blood vessel path module 110 as a blood vessel centerline.

As shown in FIG. 19, in an embodiment of the present disclosure, the single-layer mesh model structure 2 further comprises: a triangular meshing unit 21, a N-gon meshing unit 22 and a single-layer mesh model unit 23 connected in sequence. The single-layer mesh model unit 23 is connected with the blood vessel mathematical model structure 3. The triangular meshing unit 21 is connected with the three-dimensional modeling unit 500, and the triangular meshing unit is configured to perform meshing using a triangle as the smallest unit along a circumferential surface of the three-dimensional model of the blood vessel having the stenotic lesion section. The N-gon meshing unit 22 is configured to sequentially combine and convert every N triangles into a N-gon to form an initial N-gon mesh. The single-layer mesh model unit 23 is configured to remove connection lines inside each N-gon in the initial N-gon mesh to form a single-layer N-gon mesh model, where N≥6.

The present disclosure provides a coronary artery analysis system, comprising the above device for synthesizing a mathematical model of a blood vessel.

The present disclosure provides a computer storage medium, and the aforementioned method for synthesizing a mathematical model of a blood vessel having a stenotic lesion section for fluid dynamics analysis is implemented when a computer program is executed by a processor.

A person skilled in the art knows that various aspects of the present disclosure can be implemented as a system, a method, or a computer program product. Therefore, each aspect of the present disclosure can be specifically implemented in the following forms, namely: complete hardware implementation, complete software implementation (including firmware, resident software, microcode, etc.), or a combination of hardware and software implementations, which here can be collectively referred to as "circuit", "module" or "system". In addition, in some embodiments, various aspects of the present disclosure may also be implemented in the form of a computer program product in one or more computer-readable media, and the computer-readable medium contains computer-readable program code. Implementation of a method and/or a system of embodiments of the present disclosure may involve performing or completing selected tasks manually, automatically, or a combination thereof.

For example, hardware for performing selected tasks according to the embodiment(s) of the present disclosure may be implemented as a chip or a circuit. As software, selected tasks according to the embodiment(s) of the present disclosure can be implemented as a plurality of software instructions executed by a computer using any suitable operating system. In the exemplary embodiment(s) of the present disclosure, a data processor performs one or more tasks according to the exemplary embodiment(s) of a method and/or system as described herein, such as a computing platform for executing multiple instructions. Optionally, the data processor comprises a volatile memory for storing instructions and/or data, and/or a non-volatile memory for storing instructions and/or data, for example, a magnetic hard disk and/or movable medium. Optionally, a network connection is also provided. Optionally, a display and/or user input device, such as a keyboard or mouse, are/is also provided.

Any combination of one or more computer readable media can be utilized. The computer-readable medium may be a computer-readable signal medium or a computer-readable storage medium. The computer-readable storage medium may be, for example, but not limited to, an electrical, magnetic, optical, electromagnetic, infrared, or semiconductor system, apparatus, or device, or any combination of the above. More specific examples (non-exhaustive list) of computer-readable storage media would include the following:

Electrical connection with one or more wires, portable computer disk, hard disk, random access memory (RAM), read only memory (ROM), erasable programmable read only memory (EPROM or flash memory), optical fiber, portable compact disk read only memory (CD-ROM), optical storage device, magnetic storage device, or any suitable combination of the above. In this document, the computer-readable storage medium can be any tangible medium that contains or stores a program, and the program can be used by or in combination with an instruction execution system, apparatus, or device.

The computer-readable signal medium may include a data signal propagated in baseband or as a part of a carrier wave, which carries computer-readable program code. This data signal for propagation can take many forms, including but not limited to electromagnetic signals, optical signals, or any suitable combination of the above. The computer-readable signal medium may also be any computer-readable medium other than the computer-readable storage medium. The computer-readable medium can send, propagate, or transmit a program for use by or in combination with the instruction execution system, apparatus, or device.

The program code contained in the computer-readable medium can be transmitted by any suitable medium, including, but not limited to, wireless, wired, optical cable, RF, etc., or any suitable combination of the above.

For example, any combination of one or more programming languages can be used to write computer program codes for performing operations for various aspects of the present disclosure, including object-oriented programming languages such as Java, Smalltalk, C++, and conventional process programming languages, such as "C" programming language or similar programming language. The program code can be executed entirely on a user's computer, partly on a user's computer, executed as an independent software package, partly on a user's computer and partly on a remote computer, or entirely on a remote computer or server. In the case of a remote computer, the remote computer can be connected to a user's computer through any kind of network including a local area network (LAN) or a wide area network (WAN), or it can be connected to an external computer (for example, connected through Internet provided by an Internet service provider).

It should be understood that each block of the flowcharts and/or block diagrams and combinations of blocks in the flowcharts and/or block diagrams can be implemented by computer program instructions. These computer program instructions can be provided to the processor of general-purpose computers, special-purpose computers, or other programmable data processing devices to produce a machine, which produces a device that implements the functions/actions specified in one or more blocks in the flowcharts and/or block diagrams when these computer program instructions are executed by the processor of the computer or other programmable data processing devices.

It is also possible to store these computer program instructions in a computer-readable medium. These instructions make computers, other programmable data processing devices, or other devices work in a specific manner, so that the instructions stored in the computer-readable medium generate an article of manufacture comprising instructions for implementation of the functions/actions specified in one or more blocks in the flowcharts and/or block diagrams.

Computer program instructions can also be loaded onto a computer (for example, a coronary artery analysis system) or other programmable data processing equipment to facilitate a series of operation steps to be performed on the computer, other programmable data processing apparatus or other apparatus to produce a computer-implemented process, which enable instructions executed on a computer, other programmable device, or other apparatus to provide a process for implementing the functions/actions specified in the flowcharts and/or one or more block diagrams.

The above specific examples of the present disclosure further describe the purpose, technical solutions and beneficial effects of the present disclosure in detail. It should be understood that the above are only specific embodiments of the present disclosure and are not intended to limit the present disclosure. Within the spirit and principle of the present disclosure, any modification, equivalent replacement, improvement, etc. shall be included in the protection scope of the present disclosure.

## Claims

1. A method for synthesizing a mathematical model of a blood vessel having a stenotic lesion section for fluid dynamics analysis, **characterized by** comprising:
performing three-dimensional modeling according to a real-time diameter *Dₜ* of a blood vessel, a length L of a blood vessel centerline and a stenotic section to form a three-dimensional model of the blood vessel having a stenotic lesion section;
performing N-gon meshing along a circumferential surface of the three-dimensional model of the blood vessel having the stenotic lesion section to form a single-layer mesh model where N≥6;
performing surface layering on the single-layer mesh model to form a double-layer mesh model, that is, the mathematical model of the blood vessel.

2. The method for synthesizing a mathematical model of a blood vessel having a stenotic lesion section for fluid dynamics analysis according to claim 1, **characterized in that** a manner for performing N-gon meshing along a circumferential surface of the three-dimensional model of the blood vessel having the stenotic lesion section to form a single-layer mesh model where N≥6 comprises:
performing meshing using a triangle as a smallest unit along a circumferential surface of the three-dimensional model of the blood vessel having the stenotic lesion section;
sequentially combining and converting every N triangles into a N-gon to form an initial N-gon mesh;
removing connection lines inside each N-gon in the initial N-gon mesh to form a single-layer N-gon mesh model, where N≥6.

3. The method for synthesizing a mathematical model of a blood vessel having a stenotic lesion section for fluid dynamics analysis according to claim 2, **characterized in that** a manner for performing meshing using a triangle as the smallest unit along a circumferential surface of the three-dimensional model of the blood vessel having the stenotic lesion section comprises:
segmenting the three-dimensional model of the blood vessel having the stenotic lesion section into K segments,
performing meshing using a triangle as the smallest unit on a circumferential surface of each segment of the three-dimensional model of the blood vessel.

4. The method for synthesizing a mathematical model of a blood vessel having a stenotic lesion section for fluid dynamics analysis according to claim 2, **characterized in that** the triangle that uses as the smallest unit is an isosceles triangle.

5. The method for synthesizing a mathematical model of a blood vessel having a stenotic lesion section for fluid dynamics analysis according to claim 1, **characterized in that** a manner for performing surface layering on the single-layer mesh model to form a double-layer mesh model, that is, the mathematical model of the blood vessel, comprises:
acquiring a blood vessel wall thickness h;
performing three-dimensional modeling according to the blood vessel wall thickness h, a starting diameter *D_{starting}* of the blood vessel, an ending diameter *D_{ending}* of the blood vessel and the length L of the blood vessel centerline to form a truncated cone three-dimensional model on an inner surface or an outer surface of the single-layer mesh model;
performing N-gon meshing along a circumferential surface of the truncated cone three-dimensional model according to a manner for acquiring the single-layer mesh model, to form another single-layer mesh model;
forming a double-layer mesh model, that is, the mathematical model of the blood vessel, by using two layers of the single-layer mesh model and the blood vessel wall thickness h.

6. The method for synthesizing a mathematical model of a blood vessel having a stenotic lesion section for fluid dynamics analysis according to claim 1, **characterized in that** a manner for performing three-dimensional modeling according to a real-time diameter *Dₜ* of a blood vessel, a length L of a blood vessel centerline and a stenotic section to form a three-dimensional model of the blood vessel having a stenotic lesion section comprises:
acquiring two-dimensional coronary artery angiogram images of at least two body positions;
obtaining a real-time diameter *Dₜ* of the blood vessel and the length L of a straightened blood vessel centerline according to the two-dimensional coronary artery angiogram images;
performing three-dimensional modeling according to the *Dₜ* and L to form a truncated cone three-dimensional model;
acquiring the stenotic section of the coronary artery;
projecting the stenotic section onto the truncated cone three-dimensional model correspondingly to obtain a three-dimensional model of the blood vessel having a stenotic lesion section.

7. The method for synthesizing a mathematical model of a blood vessel having a stenotic lesion section for fluid dynamics analysis according to claim 6, **characterized in that** a manner for obtaining a real-time diameter *Dₜ* of the blood vessel and the length L of a straightened blood vessel centerline according to the two-dimensional coronary artery angiogram images comprises:
extracting a blood vessel centerline from the two-dimensional coronary artery angiogram image of each body position along a direction from an inlet of the coronary artery to an end of the coronary artery;
obtaining an image of a straightened blood vessel according to the two-dimensional coronary artery angiogram image and the blood vessel centerline;
obtaining a straightened blood vessel contour line according to the straightened blood vessel centerline and the image of the straightened blood vessel;
acquiring geometric information of the straightened blood vessel, comprising: the real-time diameter *Dₜ* of the blood vessel, and the length of the straightened blood vessel centerline, that is, the length L of the centerline of the straightened blood vessel.

8. The method for synthesizing a mathematical model of a blood vessel having a stenotic lesion section for fluid dynamics analysis according to claim 6, **characterized in that** a manner for performing three-dimensional modeling according to the *Dₜ* and *L* to form a truncated cone three-dimensional model comprises:
performing three-dimensional modeling according to the geometric information, the centerline and the contour line to obtain a three-dimensional model of the blood vessel having a stenotic lesion section;
acquiring a starting diameter *D_{starting}* of the blood vessel and an ending diameter *D_{ending}* of the blood vessel from the real-time diameter *Dₜ* of the blood vessel;
performing three-dimensional modeling according to the *D_{starting}, D_{ending}* and *L* to form a truncated cone three-dimensional model.

9. The method for synthesizing a mathematical model of a blood vessel having a stenotic lesion section for fluid dynamics analysis according to claim 6, **characterized by**, after acquiring two-dimensional coronary artery angiogram images of at least two body positions, and before obtaining a starting diameter *D_{starting}* of the blood vessel and an ending diameter *D_{ending}* of the blood vessel from the real-time diameter *Dₜ* of the blood vessel and the length *L* of a straightened blood vessel centerline according to the two-dimensional coronary artery angiogram images, further comprising:
acquiring a blood vessel segment of interest from the two-dimensional coronary artery angiogram image;
picking up a starting point and an ending point of the blood vessel segment of interest;
segmenting a blood vessel partial area image corresponding to the starting point and the ending point from the two-dimensional coronary artery angiogram image.

10. The method for synthesizing a mathematical model of a blood vessel having a stenotic lesion section for fluid dynamics analysis according to claim 9, **characterized in that** a manner for segmenting a blood vessel partial area image corresponding to the starting point and the ending point from the two-dimensional coronary artery angiogram image further comprises:
picking up at least one seed point of the blood vessel segment of interest;
segmenting the two-dimensional angiogram image between two adjacent points of the starting point, the seed point and the ending point, respectively, to obtain at least two blood vessel partial area images.

11. The method for synthesizing a mathematical model of a blood vessel having a stenotic lesion section for fluid dynamics analysis according to claim 7, **characterized in that** a manner for extracting a blood vessel centerline from the two-dimensional coronary artery angiogram image of each body position along a direction from an inlet of the coronary artery to an end of the coronary artery comprises:
performing image enhancement processing for the blood vessel partial area images to obtain a sharply-contrasting rough image of the blood vessel;
meshing the rough image of the blood vessel, and extracting at least one blood vessel path line along a direction from the starting point to the ending point;
selecting one blood vessel path line as the blood vessel centerline.

12. The method for synthesizing a mathematical model of a blood vessel having a stenotic lesion section for fluid dynamics analysis according to claim 11, **characterized in that** a manner for meshing the rough image of the blood vessel, and extracting at least one blood vessel path line along a direction from the starting point to the ending point comprises:
meshing the rough image of the blood vessel;
along a blood vessel extension direction from the starting point to the ending point, searching for a point having a shortest path in time with the starting point as a second point from intersecting points of surrounding n meshes, and searching for a point having a shortest path in time with the second point as a third point from intersecting points of surrounding n meshes, and repeating the above step for the third point until the shortest path in time reaches the ending point, where n is a positive integer greater than or equal to 1;
obtaining at least one blood vessel path line by connecting a line extending from the starting point to the ending point according to a searching sequence.

13. The method for synthesizing a mathematical model of a blood vessel having a stenotic lesion section for fluid dynamics analysis according to claim 12, **characterized in that** a manner for selecting one blood vessel path line as the blood vessel centerline comprises:
summing a time taken for each path line of the blood vessel extending from the starting point to the ending point if there are two or more path lines of the blood vessel;
selecting the path line of the blood vessel with a shortest time as the blood vessel centerline.

14. The method for synthesizing a mathematical model of a blood vessel having a stenotic lesion section for fluid dynamics analysis according to claim 7, **characterized in that** a manner for extracting a blood vessel centerline from the two-dimensional coronary artery angiogram image of each body position along a direction from an inlet of the coronary artery to an end of the coronary artery comprises:
performing image processing for the blood vessel partial area image to acquire an rough trend line of the blood vessel between the starting point and the ending point;
acquiring a rough edge line of the blood vessel, wherein an image between the rough edge lines of the blood vessel comprising the rough trend line of the blood vessel is a blood vessel skeleton;
extracting the blood vessel centerline from the blood vessel skeleton.

15. The method for synthesizing a mathematical model of a blood vessel having a stenotic lesion section for fluid dynamics analysis according to claim 14, **characterized in that** a manner for extracting the blood vessel centerline from the blood vessel skeleton comprises:
performing meshing for the blood vessel partial area image after image processing;
along a direction from the starting point to the ending point, according to a RGB value, searching the blood vessel skeleton for a point having a minimum difference in RGB value with the starting point from intersecting points of surrounding m meshes as a second point, searching for a point having a minimum difference in RGB value with the second point from intersecting points of surrounding m meshes as a third point, and repeating the above step for the third point until reaching the ending point, where m is a positive integer greater than or equal to 1;
obtaining at least one connection line from the starting point to the ending point according to a searching sequence;
selecting one connection line as the blood vessel centerline if there are two or more connection lines.

16. The method for synthesizing a mathematical model of a blood vessel having a stenotic lesion section for fluid dynamics analysis according to claim 15, **characterized in that** a manner for obtaining a straightened blood vessel contour line according to the straightened blood vessel centerline and the image of the straightened blood vessel comprises:
setting a threshold *D_{threshold}* for a diameter of the blood vessel on the image of the straightened blood vessel;
generating a preset contour line of the blood vessel on both sides of a centerline of the straightened blood vessel according to the *D_{threshold}*;
making the preset contour line of the blood vessel step-by-step approach the center line of the straightened blood vessel to acquire a straightened blood vessel contour line.

17. The method for synthesizing a mathematical model of a blood vessel having a stenotic lesion section for fluid dynamics analysis according to claim 6, **characterized in that** a manner for acquiring the stenotic section and the stenotic point comprises:
simulating and generating a smooth curve of a normal blood vessel according to a set extension trend of the normal blood vessel, as well as the real-time diameter *Dₜ* of the blood vessel, the length L of the centerline of the straightened blood vessel;
comparing the smooth curve of the normal blood vessel generated by simulation with a smooth curve formed by length *L*-diameter *Dₜ* of a patient's actual centerline of the straightened blood vessel to acquire a stenotic lesion section;
in the stenotic lesion section, picking a point A with the minimum diameter of the smooth curve formed by the length *L*-diameter *Dₜ* of the patient's actual centerline, and making the point A with the minimum diameter as a stenotic point of the blood vessel segment.

18. A device for synthesizing a mathematical model of a blood vessel, used for the method for synthesizing a mathematical model of a blood vessel having a stenotic lesion section for fluid dynamics analysis according to any one of claims 1 to 17, **characterized by** comprising: a three-dimensional blood vessel model structure, a single-layer mesh model structure and a blood vessel mathematical model structure connected in sequence; the blood vessel mathematical model structure being connected with the three-dimensional model structure;
the three-dimensional blood vessel model structure being configured to perform three-dimensional modeling according to a real-time diameter *Dₜ* of a blood vessel, a length L of a blood vessel centerline and a stenotic section, to form a three-dimensional model of the blood vessel having a stenotic lesion section;
the single-layer mesh model structure being configured to perform N-gon meshing along a circumferential surface of the three-dimensional model of the blood vessel having the stenotic lesion section to form a single-layer mesh model, where N ≥6;
the blood vessel mathematic model structure being configured to perform surface layering for the single-layer mesh model to form a double-layer mesh model, that is, a mathematical model of the blood vessel.

19. A coronary artery analysis system, **characterized by** comprising: the device for synthesizing a mathematical model of a blood vessel according to claim 18.

20. A computer storage medium, **characterized in that** the method for synthesizing a mathematical model of a blood vessel having a stenotic lesion section for fluid dynamics analysis according to any one of claims 1 to 17 is implemented when a computer program is executed by a processor.
